# EUROPEAN PATENT SPECIFICATION

(11) **EP 2 934 488 B1**
(45) Date of publication and mention of the grant of the patent: **17.10.2018**
(21) Application number: 13826632.5
(22) Date of filing: 19.12.2013
(51) Int. Cl.: A61K 9/16, A61K 9/20, A61K 31/4184, A61K 31/4422

(54) **A PHARMACEUTICAL COMPOSITION CONTAINING CANDESARTAN CILEXETIL AND AMLODIPINE**
PHARMAZEUTISCHE ZUSAMMENSETZUNG MIT CANDESARTAN-CILEXETIL UND AMLODIPIN
COMPOSITION PHARMACEUTIQUE CONTENANT DU CANDÉSARTAN CILEXETIL ET DE L'AMLODIPINE

(30) Priority: 21.12.2012 PL 40219112
(43) Date of publication of application: 28.10.2015
(73) Proprietor: Adamed Pharma S.A., 05-152 Czosnow (PL)
(72) Inventor: CIEPLUCHA, Agnieszka, PL-05-825 Opypy (PL)
(74) Representative: Sitkowska, Jadwiga
(86) International application number: PCT/IB2013/061138
(87) International publication number: WO 2014/097209

(56) References cited:
- WO-A1-2009/121871
- CN-A- 101 966 181
- CN-A- 102 670 603
- CN-A- 102 688 236
- CN-A- 102 743 381

## Description

### Field of the invention

The invention relates to a combination pharmaceutical composition comprising candesartan cilexetil and amlodipine or its pharmaceutically acceptable salt, a process for manufacturing of said composition, a unit dosage form containing said composition, and to the use of said composition in therapies related to hypertension.

Candesartan cilexetil is the INN for 2-{[(cyclohexyloxy)carbonyl]oxy}ethyl 2-ethoxy-1-{[2'-(1H-tetrazol-5-yl)biphenyl-4-yl]methyl}-1H-benzimidazole-7-carboxylate.

Candesartan is an atypical antagonist of angiotensin II receptor with specific selectivity against AT1 receptors and was disclosed for the first time in EP459136A. Cilexetil ester of candesartan as a prodrug, and upon absorption in the gastrointestinal tract is hydrolyzed to candesartan. Candesartan cilexetil is indicated for the treatment of essential hypertension in adults and for the treatment of adult patients with heart failure and impaired left ventricular systolic function (left ventricular ejection fraction ≤ 40%) as adjunct therapy in the treatment with angiotensin converting enzyme inhibitors (ACE inhibitors) or where they are not tolerated.

Amlodipine is the INN for 3-ethyl 5-methyl (4RS)-2-[(2-amino-ethoxy)-methyl]-6-methyl-1,4-dihydropyridine-3,5-dicarboxylate. It is a long-acting calcium channel blocker, disclosed for the first time in EP89167A and used in commercial preparations as a salt with benzenesulfonic acid (EP244944B1). Approved therapeutic indications for amlodipine are hypertension, chronic stable angina and vasospastic angina (Prinzmetal type). Amlodipine benzenesulfonate is slightly soluble in water, and its absolute bioavailability is in a range of 64-90%.

Hypertension is one of the most serious health problems in the world. It is considered a major mortality risk factor and the most common risk factor for cardiovascular diseases. Clinical trials have shown a limited efficacy of traditionally used monotherapy.

The use of a combination of two medicinal substances from different therapeutic groups acting by different pathophysiological mechanisms enables to achieve a better antihypertensive effect and more effective protection against organ damages observed in hypertension. In addition, the use of combination products is more effective than monotherapy because it is possible to simplify the treatment regimens and to increase patients compliance as well as to reduce total costs of therapy and patients convenience.

However, the preparation of combinations compositions of active substances is associated with major problems because of the need to ensure the stability of such compositions, and the appropriate size of unit dosage forms, while maintaining the desirable characteristics of the composition, such as content uniformity, hardness (tablet), dimensions of the dosage unit, etc.

Both candesartan cilexetil, and amlodipine and its pharmaceutically acceptable salts, because of the physical and chemical properties, require specific conditions for manufacturing of the formulation and the use of stabilizing excipients, or at least excipients not promoting degradation of these active substances.

One of the main problems with the oral administration of candesartan cilexetil is low bioavailability and poor solubility of this active substance. It is estimated that only 15-40% of candesartan included in commercial formulations is physiologically available and therefore active. Another problem is the sensitivity of the molecule that can undergo hydrolysis and dealkylation.

In the prior art several solutions to the above problems have been proposed. For example, in EP546358A1 to stabilize the composition of candesartan cilexetil oleaginous substances such as polyethylene glycol are used. WO2009/121871 describes a composition comprising candesartan cilexetil and a copolymer of polyvinyl alcohol and polyethylene glycol, which provides the desired stability and bioavailability of candesartan. WO2008/065097 describes stabilizing effect of substances such as diethylene glycol monoethyl ether, DL-panthenol and potassium palmitate, EP1985287A the influence of oil additives, and EP2050440A the use of certain plasticizers to stabilize the candesartan cilexetil.

Amlodipine, particularly in the form of a salt with benzenesulfonic acid, is described as "highly hygroscopic", which can lead to its degradation (WO2004075825). In the molecule of amlodipine hydrolysis / transesterification of the ester groups or the oxidation of the dihydropyridine ring may occur. Additionally, amlodipine as an amine compound shows incompatibility with some commonly-used excipients such as lactose and polyethylene glycol. Amlodipine composition stabilized with colloidal silica is disclosed in WO/0351364.

Furthermore, it was observed during formulation tests that powders of both candesartan cilexetil and amlodipine benzenesulfonate are susceptible to static electricity and exhibit poor flow properties. Such physical properties usually cause problems with uniformity of active ingredient content in the composition and technological problems during tableting or encapsulation.

In addition to stability, bioequivalence of a combination composition with respective compositions comprising single active substances is essential.

Certain combination compositions comprising candesartan cilexetil and amlodipine benzenesulfonate are described in the prior art.

EP2165702A discloses a composition of candesartan cilexetil and amlodipine benzenesulfonate, prepared by wet granulation method with water as a granulation liquid. Amlodipine can be added either to the granulation liquid or to the powder mixture and wet granulated, or added to the granulation product. The composition comprises sodium docusate as a surface active agent.

EP2106789A discloses a composition of candesartan cilexetil and amlodipine benzenesulfonate, comprising a graft copolymer of polyvinyl alcohol with polyethylene glycol. The copolymer is used to chemically and physically stabilize the composition. WO2010/126168 discloses a combination composition of candesartan and amlodipine, in which both active substances are wet granulated separately and then both granulates are mixed together and tabletted.

CN102764258 discloses combination composition comprising candesartan and amlodipine, wherein the ratio of candesartan to PEG 6000 is in a range of from 1:0.5 to 1:0.8. However, this composition is prepared in the form of a simple mixture, and the amount of polyethylene glycol is not sufficient to stabilize the active ingredients which are not separated from each other.

CN102670603 discloses combination composition comprising candesartan and amlodipine, wherein candesartan is subjected to wet granulation prior to compression with amlodipine. However, this composition lacks stabilizing glycols.

CN102688236 discloses combination composition comprising candesartan, amlodipine and PEG 6000 in the amount of 2-4% with respect to the total weight of the composition. In this composition candesartan and amlodipine before tabletting are in the form of a powder blend. The contact between active ingredients as well as between certain excipients is not limited. In consequence, amlodipine is exposed to direct contact with factors that may cause its degradation, i.e. to the contact with polyethylene glycol, which results in increased impurity D, and to the contact with lactose, which causes formation of colored impurities (Maillard reaction). Additionally, the range of polyethylene glycol contents significantly and adversely affects bioavailability of the composition and in consequence bioequivalence with the active ingredients, especially with candesartan, is difficult to obtain.

As mentioned above, due to the properties of the active substances as well as therapeutic indication of the combination composition comprising candesartan cilexetil and amlodipine, it is desirable during manufacture process to avoid conditions (humidity, temperature) as well as the use of excipients, the nature or amount of which could have a negative effect on the composition by promoting the degradation of the active substances or which otherwise would be undesirable in the composition, such as for example excipients in the form of sodium salts which are deleterious in the preparations for the treatment of hypertension.

The aim of the invention is to provide a combination pharmaceutical composition comprising candesartan cilexetil and amlodipine or a pharmaceutically acceptable salt thereof, wherein both active substances remain stable, both in the process of preparation and during the storage, and to provide a technologically simple process of the preparation of said composition wherein active substances are not exposed to the factors that cause their degradation. Furthermore, the purpose of the present invention is to provide a combination composition with suitable bioavailability, as close as possible to the bioavailability of separate formulations containing respective single active ingredients.
The object of the present invention is achieved by the invention described herein.

The object of the present invention is therefore a combination pharmaceutical composition in the form of a uniform solid blend consisting of:
a) a granulate comprising candesartan cilexetil, polyethylene glycol and excipients selected from the group consisting of a filler, a disintegrant, a binder and mixtures thereof, wherein the weight ratio of candesartan cilexetil to polyethylene glycol is in the range from 5:1 to 2:1, and
b) an extragranular phase which is a powder blend comprising amlodipine or a pharmaceutically acceptable salt thereof and excipients selected from the group consisting of a filler, lubricant and mixtures thereof, or from the group consisting of a disintegrant, a lubricant and mixtures thereof.

The term "combination pharmaceutical composition", as used herein, refers to a pharmaceutical composition comprising a combination of the two active ingredients contained in a single dosage form.

The term "powder blend", as used herein, refers to a powder mixture obtained by simple mixing without any operations of granulation, compaction, slugging, and the like, either before simple mixing or after simple mixing.

The excipients in the granulate a) are selected from the group consisting of a filler, a disintegrant, a binder, and mixtures thereof.

Preferably, the excipients in the granulate a) are a mixture comprising 80.0-90.0% by weight of the filler, 0-3.0% by weight of the disintegrant, and 2.5-12,0% by weight of the binder, calculated on the total weight of the granulate a).

More preferably, the excipients in the granulate a) are the mixture comprising 83.0-90.0% by weight of the filler, most preferably 85.0-90.0%; 0.5 - 2.5% by weight of the disintegrant, most preferably 1.0% or 2.0%; and 5.0-8.0% by weight of the binder, most preferably 6.0% - 6.5%, calculated on the total weight of the granulate a).

The filler in the granulate a) and in the extragranular phase b) of the composition according to the invention can for example be selected from the group consisting of oligosaccharides such as anhydrous lactose or lactose monohydrate, sucrose, polysaccharides such as microcrystalline cellulose, starch, pregelatinized starch, and mixtures thereof, as well as co-processed mixtures thereof, such as microcrystalline cellulose coated with the colloidal silica.

In a preferred embodiment of the pharmaceutical composition of the invention, the filler in the granulate a) is selected from the group consisting of anhydrous lactose, lactose monohydrate, microcrystalline cellulose, starch, pregelatinized starch, and mixtures thereof.

In a preferred variant of the above embodiment of the pharmaceutical composition of the invention, the filler in the granulate a) is a mixture of monohydrate lactose and starch, and the weight ratio of candesartan cilexetil to polyethylene glycol is 4.8:1.0 or 2.4:1.0 .

In another preferred variant of the above embodiment of the pharmaceutical composition of the invention, the filler in the granulate a) is a mixture of starch, pregelatinized starch and microcrystalline cellulose, and the weight ratio of candesartan cilexetil to polyethylene glycol is 4.8:1.0 or 2.4:1.0.

The disintegrant in the granulate a) of the composition of the invention can be selected from the group consisting of carboxymethylcellulose calcium salt, carboxymethylcellulose sodium salt, carboxymethyl starch sodium salt, microcrystalline cellulose, colloidal anhydrous silica, crospovidone, for example, type A crospovidone (Polyplasdone XL ®), and maize starch.

In a preferred embodiment of the pharmaceutical composition of the invention the disintegrant in the granulate a) is carboxymethylcellulose calcium salt.

The binder in the granulate a) of the composition of the invention can be selected from the group consisting of cellulose derivatives such as hydroxypropylcellulose, hydroxypropylmethylcellulose, methylcellulose, carboxymethylcellulose, hydroxycellulose and hydroxyethylcellulose, polyvinyl alcohol, polyvinyl pyrrolidone, and mixtures thereof.

In a preferred embodiment of the pharmaceutical composition of the invention the binder in the granulate a) is a hydroxypropylcellulose.

In a particularly preferred embodiment of the pharmaceutical composition of the invention the binder in the granulate a) is a mixture of hydroxypropylcelluloses, preferably of at least two types of hydroxypropylcelluloses, wherein at least one of these types of hydroxypropylcelluloses is in the form of an aqueous solution. Examples of hydroxypropylcellulose derivatives, which are suitable for use in the form of an aqueous solution are those which are characterized by low dynamic viscosity in aqueous media, e.g. in water, i.e. viscosity of about 400 mPa * s, or less than 150 mPa * s measured at 25° C for 2% water solution.

Moreover, hydroxypropylcellulose derivatives, which are suitable for use in the form of a solution are characterized by a low molecular weight, preferably in the range of 50 000 -150000. Examples of such hydroxypropylcellulose derivatives are Aqualon products such as Klucel LF, Klucel EF or Klucel JF, and products of Nippon Soda such as Nisso HPC-L. Examples of hydroxypropylcellulose derivatives that are suitable for use in a preferred embodiment of the pharmaceutical composition of the invention in admixture with hydroxypropylcellulose derivative in the form of a solution are hydroxypropylcelluloses with fine particle size. Examples of such hydroxypropylcelluloses are Klucel EF grade with regular particle size or alternatively, grades EXF or EXAF marketed by Aqualon. Preferable are hydroxypropylcelluloses with low viscosities in water media i.e. viscosities in a range of 300-600 mPa*s, most preferably with viscosities below 100 mPa*s measured at 25°C for 2% water solution. Most preferable as a binder is a mixture of hydroxypropylcellulose Klucel LF in the form of an aqueous solution and hydroxypropylcellulose Klucel EXF in the form of a dry powder.

The use of the mixture of hydroxypropylcelluloses ensures desired bioavailability and stability of the complex composition and addition of additional granulation liquid in a separate step is not required.

Hydroxypropylcellulose in the form of a solution can be added to a mixture of dry ingredients, and then wet granulation process be performed. In such a case additional granulation liquid such as water or other solvent is not added.

The contents of a disintegrant and a filler in the granulate a), is dependent on the amount/percentage of the active ingredient candesartan cilexetil. The percentage of candesartan cilexetil, in turn, varies depending on the desired amount of active ingredient per dosage unit (potency).

The extragranular phase b) is a powder blend comprising amlodipine or a pharmaceutically acceptable salt thereof and excipients.

In one variant, the excipients in the extragranular phase b) are selected from the group consisting of a filler, a lubricant, and mixtures thereof.

In a preferred embodiment of this first variant, excipients in the extragranular phase b) are a mixture of 80.0-95.0% by weight of the filler and 1.0-3.0% by weight of the lubricant.

In a more preferred embodiment of the first variant, the excipients in the extragranular phase b) are the mixture of 88.0% by weight of the filler and 1.8% by weight of the lubricant.

In another preferred embodiment of the first variant, the excipients in the extragranular phase b) are the mixture of 93.0% by weight of the filler and 1.8% by weight of the lubricant.

In a second variant, the excipients in the extragranular phase b) are a mixture of a disintegrant and a lubricant.

In a preferred embodiment of this second variant, the excipients in the extragranular phase b) are a mixture of 22.0-66.0% by weight of the disintegrant and 5.0-15.0% by weight of the lubricant.

Most preferably in this second variant, the extragranular phase b) contains 42.0-44.0% by weight of the disintegrant and 10-13% of the lubricant.

The contents of a lubricant and a filler in the extragranular phase b) is dependent on the amount/percentage of the active ingredient amlodipine or a pharmaceutically acceptable salt thereof. This percentage, in turn, varies depending on the desired amount of amlodipine per dosage unit.

Thus, in one embodiment of the first variant, for the preparation of a dosage unit containing a dose of 10 mg of amlodipine, especially in the form of benzenesulfonate salt, excipients in the extragranular phase b) are preferably a mixture of 88 % by weight of a filler and 1.8% by weight of a lubricant. In turn, for the preparation of a dosage unit containing a dose of 5 mg of amlodipine, especially in the form of benzenesulfonate salt, excipients in the extragranular phase b) are preferably a mixture of 93.0 % by weight of a filler and 1.8 % by weight of a lubricant.

The filler in the extragranular phase b) in this embodiment of the first variant is chosen from the group as described above for the granulate a).

Preferably, the filler in extragranular phase b) in this embodiment of the first variant is microcrystalline cellulose.

The lubricant in the extragranular phase b) of the composition of the invention in this embodiment of the first variant may be selected from the group consisting of magnesium stearate, sodium stearyl fumarate, calcium stearate, and hydrogenated vegetable oil, for example hydrogenated castor oil. Preferably, the lubricant in the extragranular phase b) is magnesium stearate.

In this preferred embodiment of the first variant of the pharmaceutical composition of the invention the weight ratio of the granulate a) to the extragranular phase b) is in the range from 1.0:1.0 to 2.0:1.0.

More preferably, in this preferred embodiment of the first variant the weight ratio of the granulate a) to the extragranular phase b) is 1.5:1.0.

In an even more preferred embodiment of the second variant of the pharmaceutical composition of the invention, for the preparation of a dosage unit containing a dose of 10 mg of amlodipine, especially in the form of benzenesulfonate, excipients in the extragranular phase b) are preferably a mixture of 43.7% by weight of a disintegrant and 10.0% by weight of a lubricant. In the pharmaceutical composition of the invention for the preparation of a dosage unit containing a dose of 5 mg of amlodipine, especially in the form of benzenesulfonate, excipients in the extragranular phase b) are preferably a mixture of 56.9% by weight of a disintegrant and 13.0% by weight of a lubricant.

The disintegrant in the extragranular phase b) in this even more preferred embodiment of the second variant can be selected from the group as described above for the granulate a).

Preferred disintegrant in the extragranular phase b) is carboxymethylcellulose calcium salt.

The lubricant in the extragranular phase b) in this even more preferred embodiment of the second variant may be selected from the group consisting of magnesium stearate, sodium stearyl fumarate, calcium stearate, and hydrogenated vegetable oil, for example hydrogenated castor oil.

Most preferably, a lubricant in the extragranular phase b) in this even more preferred embodiment of the second variant is magnesium stearate.

In this even more preferred embodiment of the second variant of the pharmaceutical composition of the invention the weight ratio of the granulate a) to the extragranular phase b) is in the range from 13.0:1.0 to 10.0:1.0.

More preferably in this embodiment of the second variant of the pharmaceutical composition of the invention the weight ratio of the granulate a) to the extragranular phase b) is 10.0:1.0 or 13.0:1.0.

For numerical values expressing the ratio of the percentage of the granulate a) to the extragranular phase b), the weight ratio of candesartan cilexetil to polyethylene glycol and the percentage of the individual excipients in the composition of the invention normal rules of rounding should be applied. For example, if in the pharmaceutical composition of the invention the weight ratio of the granulate a) to extragranular phase b) is 1.48:1.0 or 1.54:1,0, it is rounded and presented as 1.5:1.0.

Preferably, the pharmaceutically acceptable salt of amlodipine in the extragranular phase b) is amlodipine benzenesulfonate.

Processes for the preparation of both active substances are known from the prior art. Amlodipine benzenesulfonate can be prepared, for example, by the method disclosed in EP0993447B1, and candesartan cilexetil - by the method described in EP459136B1.

The invention also provides a process of the preparation of a combination pharmaceutical composition, as described above, comprising the following steps:
- mixing the components of the granulate a) to obtain a homogeneous powder blend
- wet granulation of the homogeneous blend,
- drying the obtained granulate,
- uniformizing the granulate to form granulate a),
- mixing the obtained granulate a) with the components of the extragranular phase b).

Mixing of the components of the granulate a) to obtain the homogeneous blend can be carried out by standard methods known to those skilled in the art, for example in the container of a high shear granulator. The thus obtained mixture is granulated. The granulating liquid may be an aqueous solution of binder or an aqueous solution of the binder and polyethylene glycol, or a homogeneous suspension of the active substance candesartan cilexetil in an aqueous binder solution or a homogeneous suspension of the active substance candesartan cilexetil in an aqueous solution of a binder and polyethylene glycol. All above option of the granulating liquid will provide a substantially similar effect of binding of components of the powder mixture.

Those skilled will appreciate that, alternatively, the granulation liquid can be water, but this variant that is technologically more complex because of the need to provide a source of water of a specified quality and because of the necessity to perform an additional process step of adding water and mixing it with remaining components.

The wet granulate is then subjected to a drying process, preferably a drying process in a fluidized bed, to obtain a dry granulate. The term "dry granulate" refers to the granulate, wherein the water content is approximately equal to the water content in the starting mixture of the components prior to granulation. Uniformizing the dry granulate to form the final granulate a) may be performed using conventional means known to those skilled in the art. Preferably, uniformization of the dry granulate is carried out by sizing in the oscillating or rotary granulator.

The final composition of the invention is obtained by mixing the granulate a) with the components of extragranular phase b) using the equipment and process parameters enabling the preparation of a final uniformized blend of all components, which may for example be confirmed by the analysis of uniformity of the content of the individual active substances.

The combination pharmaceutical composition according to the invention is a solid blend containing the granulate a) and the extragranular phase b).

The composition according to the invention after its preparation is used for the preparation of unit dosage forms.

Therefore, a further object of the invention is a unit dosage form comprising the pharmaceutical composition of the invention or prepared by the method according to the invention. The pharmaceutical composition of the invention is comprised in such a unit dosage form.

In particular, the unit dosage form is selected from tablets, capsules, sachets, stick-type sachets and ampoules. Preferred unit dosage forms are tablets and hard capsules, for example, gelatin or hydroxypropylmethylcellulose capsules. In a most preferred embodiment, the unit dosage forms are tablets. The tablets may be uncoated or coated.

It is obvious to one of ordinary skill that the amount of the composition of the invention, which is compressed into a tablet or encapsulated in a capsule must be sufficient to ensure adequate doses of active ingredients per dosage unit.

The size of the dosage form, preferably tablet, is dependent on the active ingredient dose and the amount of the pharmaceutical composition of the invention comprising this dose.

The amount of candesartan cilexetil is 8 or 16 mg per dosage unit of the combination pharmaceutical composition of the invention, and the amount of amlodipine (based on free base) is 5 or 10 mg per dosage unit of the combination pharmaceutical composition of the invention. Preferably, amlodipine is in the form of benzenesulfonate salt, and amounts of said salt per dosage unit are 13.87 g and 6.94 g, respectively.

Preparation of tablets and filling of capsules with composition of the invention are known technological operations in the field of pharmaceutical technology and can be performed using standard equipment.

For example, tabletting of the pharmaceutical composition of the invention can be performed using a rotary tablet press.

For example, capsules may be filled using a gravity hopper or various types of feeders, depending on the type of encapsulation machine.

The following examples illustrate the invention.

### Example 1.

Components of a combination composition per dosage unit (a tablet) are presented in Table 1.

**Table 1.**

| Components | Dose of candesartan cilexetil/amlodipine calculated as free base [mg/mg] in dosage unit | | | |
|---|---|---|---|---|
| | 8/5 | 16/10 | 8/10 | 16/5 |
| *Components of the granulate [mg]* | | | | |
| Candesartan cilexetil | 8 | 16 | 8 | 16 |
| Pregelatinized starch | 48.6 | 97.2 | 105.2 | 97.2 |
| Microcrystalline cellulose | 24.75 | 49.5 | 49.5 | 49.5 |
| Starch | 9.9 | 19.8 | 19.8 | 19.8 |
| Carboxymethylcellulose calcium | 2.48 | 4.95 | 4.95 | 4.95 |
| Polyethylene glycol PEG 8000 | 3.3 | 6.6 | 6.6 | 6.6 |
| Hyd roxypropylcellu lose | 2.97 | 5.95 | 5.95 | 5.95 |
| Purified water | q.s. | q.s. | q.s. | q.s. |
| *Weight of the granulate* | 100.0 | 200.0 | 200.0 | 200.0 |

| *Components of the extragranular phase [mg]* | | | | |
|---|---|---|---|---|
| Amlodipine benzenesulfonate | 6.94 | 13.87 | 13.87 | 6.94 |
| Microcrystalline cellulose | 56.9 | 113.8 | 113.8 | 120.73 |
| Magnesium stearate | 1.16 | 2.33 | 2.33 | 2.33 |
| *Weight of the extragranular phase* | 65.0 | 130.0 | 130.0 | 130.0 |
| *Total weight* **[mg]** | 165.0 | 330.0 | 330.0 | 330.0 |

Tablet cores containing candesartan cilexetil and amlodipine benzenesulfonate are prepared by compression of the blend comprising the granulate of candesartan cilexetil with admixed extragranular phase comprising amlodipine benzenesulfonate.

The dry components of the granulate. i.e. candesartan cilexetil, pregelatinized starch, microcrystalline cellulose, starch, carboxymethylcellulose calcium salt, polyethylene glycol and hydroxypropylcellulose in amounts per one tablet as shown in the Table 1 above were mixed in a high shear granulator until a homogenous blend was obtained, then wetted with purified water and granulated. The wet granulate were then subjected to fluidized bed drying process to obtain a moisture level corresponding approximately to the starting moisture content of the dry ingredients mixture.

The dry granulate was then uniformized by sizing using an oscillating granulator. The resulting granulate comprising candesartan cilexetil was mixed with the extragranular phase components: amlodipine benzenesulfonate, microcrystalline cellulose and magnesium stearate in a standard blending process in a bin, and then were tabletted using a rotary tablet machine with biconvex punches of 8 mm diameter for the dose of 8/5 and 10 mm diameter for the remaining doses.

### Example 2.

Components of a combination composition per dosage unit (a tablet) are presented in Table 2.

**Table 2.**

| *Component* | Dose of candesartan cilexetil/amlodipine calculated as free base [mg/mg] in dosage unit | | | |
|---|---|---|---|---|
| | 8/5 | 16/10 | 8/10 | 16/5 |
| *Components of the granulate [mg]* | | | | |
| Candesartan cilexetil | 8 | 16 | 8 | 16 |
| Lactose monohydrate | 70.0 | 140.1 | 148.1 | 140.1 |
| Starch | 14.9 | 29.7 | 29.7 | 29.7 |
| Carboxymethylcellulose calcium | 2.48 | 4.95 | 4.95 | 4.95 |
| Polyethylene glycol PEG 8000 | 1.65 | 3.3 | 3.3 | 3.3 |
| Hydroxypropylcellulose | 2.97 | 5.95 | 5.95 | 5.95 |
| Purified water | q.s. | q.s. | q.s. | q.s. |
| *Weight of the granulate* | 100.0 | 200.0 | 200.0 | 200.0 |

| *Components of extragranular phase[mg]* | | | | |
|---|---|---|---|---|
| Amlodipine benzenesulfonate | 6.94 | 13.87 | 13.87 | 6.94 |
| Microcrystalline cellulose | 56.9 | 113.8 | 113.8 | 120.73 |
| Magnesium stearate | 1.16 | 2.33 | 2.33 | 2.33 |
| *Weight of the extragranular phase* | 65.0 | 130.0 | 130.0 | 130.0 |
| *Total weight of the dosage form* **[mg]** | 165.0 | 330.0 | 330.0 | 330.0 |

Tablet cores containing candesartan cilexetil and amlodipine benzenesulfonate were prepared by compression of the blend comprising the granulate of candesartan cilexetil with admixed extragranular phase comprising amlodipine benzenesulfonate.

The dry components of the granulate, i.e. candesartan cilexetil, lactose monohydrate, starch, carboxymethylcellulose calcium salt, polyethylene glycol and hydroxypropylcellulose in the amounts per one tablet as shown in the Table 2 above were mixed and granulated. The wet granulate were then subjected to fluidized bed drying process to obtain a moisture level corresponding approximately to the starting moisture content of the dry ingredients mixture.

The dry granulate was then uniformized by sizing using an oscillating granulator. The resulting granulate comprising candesartan cilexetil was mixed with the extragranular phase components: amlodipine benzenesulfonate, microcrystalline cellulose and magnesium stearate in a standard blending process in a bin, and then tabletted using a rotary tablet machine with biconvex punches of 8 mm diameter for the dose of 8/5 and 10 mm diameter for the remaining doses.

### Example 3.

Components of a combination composition per dosage unit (a capsule) are presented in Table 3.

**Table 3.**

| *Component* | Dose of candesartan cilexetil/amlodipine calculated as free base [mg/mg] in dosage unit: 16/10 |
|---|---|
| *Components of the granulate [mg]* | |
| Candesartan cilexetil | 16 |
| Pregelatinized starch | 90.0 |
| Microcrystalline cellulose | 39.35 |
| Starch | 20.0 |
| Carboxymethylcellulose calcium | 4.95 |
| Polyethylene glycol PEG 8000 | 6.7 |
| Hydroxypropylcellulose | 3.0 |
| Purified water | q.s. |
| *Weight of the granulate* | 180.0 |

| *Components of extragranular phase[mg]* | |
|---|---|
| Amlodipine benzenesulfonate | 13.87 |
| Microcrystalline cellulose | 133.8 |
| Magnesium stearate | 2.33 |
| *Weight of the extragranular phase* | 150 |
| *Total weight* **[mg]** | 330.0 |

Capsules containing candesartan cilexetil and amlodipine benzenesulfonate were prepared by encapsulation of a blend comprising granulate of candesartan cilexetil with admixed extragranular phase comprising amlodipine benzenesulfonate.

The dry components of the granulate: candesartan cilexetil, pregelatinized starch, microcrystalline cellulose, carboxymethylcellulose calcium salt, polyethylene glycol and hydroxypropylcellulose in the amounts per one capsule as shown in the Table 3 above were mixed in a high shear granulator until a homogenous blend was obtained, then wetted with purified water and granulated. The wet granulate were then subjected to fluidized bed drying process to obtain a moisture level corresponding approximately to the starting moisture content of the dry ingredients mixture.

The dry granulate was then uniformized by sizing using an oscillating granulator. The resulting granulate comprising candesartan cilexetil was mixed with the extragranular phase components: amlodipine benzenesulfonate, microcrystalline cellulose and magnesium stearate in a standard blending process in a bin. The thus obtained blend was used to fill hard gelatin capsules of size 0, using the encapsulating machine MG COMPACT from MG2 company.

### Example 4.

Components of a combination composition per dosage unit (a tablet) are presented in Table 4.

**Table 4**

| *Component* | Dose of candesartan cilexetil/amlodipine calculated as free base [mg/mg] in dosage unit | | | | | |
|---|---|---|---|---|---|---|
| | 16/10 variant A | 16/5 variant A | 16/10 variant B | 16/5 variant B | 8/10 variant A | 8/10 variant B |
| *Components of the granulate [mg]* | | | | | | |
| Candesartan cilexetil | 16 | 16 | 16 | 16 | 8 | 8 |
| Lactose monohydrate | 203.9 | 203.9 | 193.2 | 193.2 | 211.9 | 201.2 |
| Corn starch | 52.8 | 52.8 | 65.0 | 65.0 | 52.8 | 65.0 |
| Hydroxypropylcellulose Klucel LF/ in solution | 4.5 | 4.5 | 5.5 | 5.5 | 4.5 | 5.5 |
| Hydroxypropylcellulose Klucel EXF | 13.5 | 13.5 | 14.0 | 14.0 | 13.5 | 14.0 |
| Polyethylene glycol PEG 8000 | 3.3 | 3.3 | 3.3 | 3.3 | 3.3 | 3.3 |
| Carboxymethylcellulose calcium salt | 6.0 | 6.0 | 3.0 | 3.0 | 6.0 | 3.0 |
| *Weight of the granulate* | 300.0 | | | | | |

| *Components of the extragranular phase [mg]* | | | | | | |
|---|---|---|---|---|---|---|
| Amlodipine benzenesulfonate | 13.87 | 6.94 | 13.87 | 6.94 | 13.87 | 13.87 |
| Carboxymethylcellulose calcium | 13.1 | 13.1 | 13.1 | 13.1 | 13.1 | 13.1 |
| Magnesium stearate | 3.0 | 3.0 | 3.0 | 3.0 | 3.0 | 3.0 |
| *Weight of the extragranular phase* | 30 | 23.1 | 30 | 23.1 | 30 | 30 |
| *Total weight* **[mg]** | 330.0 | 323.1 | 330.0 | 323.1 | 330.0 | 330.0 |

Tablet cores containing candesartan cilexetil and amlodipine benzenesulfonate were prepared by compression of the blend comprising the granulate of candesartan cilexetil with admixed extragranular phase comprising amlodipine benzenesulfonate. The dry components of the granulate: candesartan cilexetil, lactose monohydrate, corn starch, carboxymethylcellulose calcium, polyethylene glycol, and hydroxypropylcellulose Klucel EXF and solution of hydroxypropylcellulose Klucel LF in the amounts per one tablet as shown in the Table 4 above were mixed in a high shear granulator until a homogenous blend was obtained, and granulated. The wet granulate was then subjected to fluidized bed drying process to obtain a moisture level corresponding approximately to the starting moisture content of the dry ingredients mixture.

The dry granulate was then uniformized by sizing using a rotating granulator. The resulting granulate comprising candesartan cilexetil was mixed with the extragranular phase components: amlodipine benzenesulfonate, carboxymethylcellulose calcium and magnesium stearate in a standard blending process in a bin, and then tabletted using a rotary tablet machine with biconvex punches of 10 mm diameter.

### Example 5.

Stability studies at accelerated conditions for the tablets of Example 1, for doses 16/10 and 16/5 ; the tablets of Example 2, for doses 16/10 and 16/5, and for the capsules of Example 3 were performed. The compositions were analyzed after 3 months of storage at 40 °C, 75% RH in blisters Alu / Alu. The level of impurities was determined by HPLC.

The results are presented in Table 5.

**Table 5.**

| | **Tablets 16/10 of Example 1** | | **Tablets 16/5 of Example 1** | | **Tablets 16/10 of Example 2** | | **Tablets 16/5 of Example 2** | | **Capsules 16/10 of Example 3** | |
|---|---|---|---|---|---|---|---|---|---|---|
| *Amlodipine impurities (limit)* | start [%] | 3 months [%] | start [%] | 3 months [%] | start [%] | 3 months [%] | start [%] | 3 months [%] | start [%] | 3 months [%] |
| D*-AML (≤ 0,5%) | < 0.05 | 0.20 | <0.05 | 0.25 | <0.05 | <0.05 | <0.05 | <0.05 | <0.05 | 0.07 |

| *Candesartan cilexetil impurities (limit)* | | | | | | | | | | |
|---|---|---|---|---|---|---|---|---|---|---|
| B*-CAN (≤ 1.0%) | 0.09 | 0.54 | 0.10 | 0.60 | 0.09 | 0.32 | 0.09 | 0.33 | 0.09 | 0.33 |
| C*-CAN (≤ 0.2%) | n.d. | < 0.05 | n.d. | 0.06 | n.d. | <0.05 | n.d. | <0.05 | n.d. | <0.05 |
| D*-CAN (≤ 0.2%) | n.d. | 0.08 | n.d. | 0.10 | n.d. | 0.05 | n.d. | 0.05 | n.d. | n.d. |
| E*-CAN (≤ 0.5%) | <0.05 | 0.16 | <0.05 | 0.19 | <0.05 | 0.10 | <0.05 | 0.11 | <0.05 | 0.11 |
| F*-CAN (≤ 0.5%) | 0.07 | 0.33 | 0.07 | 0.36 | 0.06 | 0.2 | 0.06 | 0.2 | 0.06 | 0.24 |
| G*-CAN (≤ 0.5%) | n.d. | n.d. | n.d. | n.d. | n.d. | n.d. | n.d. | n.d. | n.d. | n.d. |
| *Max. Single unknown impurity* (≤0,5%) | <0.05 | 0.06 | 0.09 | 0.09 | 0.05 | 0.15 | 0.09 | 0.22 | <0.05 | 0.10 |
| **total impurities (≤ 3.5%)** | 0.16 | 1.40 | 0.33 | 1.9 | 0.20 | 0.89 | 0.35 | 1.1 | 0.17 | 0.86 |
| *Letter designations of impurities are consistent with nomenclature given in European Pharmacopoeia 7th Edition | | | | | | | | | | |

### Example 6.

Stability studies at accelerated conditions for the tablets of Example 4, for doses 16/10 variant A and 16/5 variant A; the tablets of Example 4. The compositions were analyzed after 3 months of storage at 40 °C, 75% RH in blisters Alu / Alu. The level of impurities was determined by HPLC.

The results are presented in Table 6

| | **Tablets 16/10 of Example 4 variant A** | | **Tablets 16/5 of Example 4 variant A** | |
|---|---|---|---|---|
| *Amlodipine impurities (limit)* | start [%] | 3 months [%] | start [%] | 3 months [%] |
| D*-AML (≤ 0.5%) | <0.05 | <0.05 | <0.05 | 0.07 |
| *Max. single unknown AML impurity* (≤ 0.5%) | <0.05 | 0.12 | <0.05 | 0.24 |
| *Candesartan cilexetil impurities (limit)* | | | | |
| B*-CAN (≤ 1.0%) | 0.10 | 0.31 | 0.11 | 0.32 |
| C*-CAN (≤ 0.2%) | n.d. | n.d. | n.d. | n.d. |
| D*-CAN (≤ 0.2%) | n.d. | 0.05 | n.d. | <0.05 |
| E*-CAN (≤ 0.5%) | <0.05 | 0.10 | <0.05 | 0.11 |
| F*-CAN (≤ 0.5%) | 0.09 | 0.24 | 0.09 | 0.24 |
| G*-CAN (≤ 0.5%) | n.d. | n.d. | n.d. | n.d. |
| *Max. single unknown CAN impurity* (≤ 0.5%) | <0.05 | <0.05 | <0.05 | <0.05 |
| **total impurities (≤ 3.5%)** | 0.19 | 0.83 | 0.20 | 1.18 |
| *Letter designations of impurities are consistent with nomenclature given in European Pharmacopoeia 7th Edition | | | | |

**Example 7.** Comparative study - influence of lack of separation of active ingredients into two phases on the stability of combination composition comprising candesartan cilexetil and amlodipine.

Tablets of the composition presented in Table 7 above with candesartan cilexetil and amlodipine benzenesulfonate incorporated in the same granulate were prepared.

**Table 7**

| *Component* | Dose of candesartan cilexetil/amlodipine calculated as free base [mg/mg] in dosage unit | |
|---|---|---|
| | 16/10 | 16/5 |
| Candesartan cilexetil | 16 | 16 |
| Amlodipine benzenesulfonate | 13.87 | 6.94 |
| Lactose monohydrate | 203.9 | 203.9 |
| Corn starch | 52.8 | 52.8 |
| Hydroxypropylcellulose Klucel LF/ in solution | 4.5 | 4.5 |
| Hydroxypropylcellulose Klucel EXF | 13.5 | 13.5 |
| Polyethylene glycol PEG 8000 | 3.3 | 3.3 |
| Carboxymethylcellulose calcium salt | 19.1 | 19.1 |
| Magnesium stearate | 3.0 | 3.0 |
| *Total weight* **[mg]** | 330.0 | 323.1 |

Tablet cores containing candesartan cilexetil and amlodipine benzenesulfonate were prepared by compression of the blend comprising the candesartan cilexetil, amlodipine benzenesulfonate and excipients. Candesartan cilexetil, amlodipine benzenesulfonate lactose monohydrate, corn starch, carboxymethylcellulose calcium, polyethylene glycol, and hydroxypropylcellulose Klucel EXF in the amounts per one tablet as shown in the Table 4 above were mixed in a high shear granulator until a homogenous blend was obtained and granulated with hydroxypropylcellulose Klucel LF water solution. The wet granulate was then subjected to fluidized bed drying process to obtain a moisture level corresponding approximately to the starting moisture content of the dry ingredients mixture.

The dry granulate was then uniformized by sizing and tabletted using a rotary tablet machine with biconvex punches of 10 mm diameter. The obtained compositions were analyzed after 3 months of storage at 40 °C, 75% RH in blisters Alu / Alu. The level of impurities was determined by HPLC. Stability studies at accelerated conditions for the obtained tablets, for doses 16/10 and 16/5 are presented in Table 8.

**Table 8**

| | **Tablets 16/10 of Ex. 7** | | **Tablets 16/5 of Ex. 7** | |
|---|---|---|---|---|
| *Amlodipine impurities (limit)* | start [%] | 3 months [%] | start [%] | 3 months [%] |
| D*-AML (≤ 0.5%) | 0.05 | 0.28 | 0.07 | 0.57 |
| *Max. single unknown AML impurity* (≤ 0.5%) | 0.05 | 0.59 | 0.05 | 1.08 |

| *Candesartan cilexetil impurities (limit)* | | | | |
|---|---|---|---|---|
| B*-CAN (≤ 1.0%) | 0.10 | 0.72 | 0.11 | 0.84 |
| C*-CAN (≤ 0.2%) | n.d. | n.d. | n.d. | n.d. |
| D*-CAN (≤ 0.2%) | n.d. | 0.05 | n.d. | 0.05 |
| E*-CAN (≤ 0.5%) | 0.05 | 0.10 | 0.05 | 0.11 |
| F*-CAN (≤ 0.5%) | 0.10 | 0.55 | 0.11 | 0.60 |
| G*-CAN (≤ 0.5%) | n.d. | n.d. | n.d. | n.d. |
| *Max. single unknown CAN impurity* (≤ 0,5%) | 0.05 | 0.65 | 0.05 | 0.45 |
| **total impurities (≤ 3.5%)** | 0.43 | 3.13 | 0.49 | 3.82 |

### Example 8

Bioequivalence studies were performed according to the 2010 Guidelines on the Investigation of Bioequivalence Doc. Ref.: CPMP/EWP/QWP/1401/98 Rev. 1/ Corr ** released by the Committee for Medicinal Products for Human use (CHMP). Pharmacokinetic studies were performed for tablets of AML/CAN prepared according to Example 4 variant A and Example 7 (comparative example). Results of the studies are presented in Tables 9-12

**Table 9. PK data for tablets AML/CAN prepared according to Example 4; requirements 80 % <T/R<125%), reference: amlodipine 10 mg.**

| Parameter | N | TEST CAN-AML 16/10 mg Example 4 variant A | | REFERENCE AML 10 mg | | RATIO |
|---|---|---|---|---|---|---|
| | | MEAN | CV (%) | MEAN | CV (%) | T/R (%) |
| AUC0-t (ng h/mL) | 16 | 113.34 | 23.60 | 112.25 | 26.86 | 100.98 |
| AUC0-∞ (ng h/mL) | 16 | 161.50 | 26.71 | 166.13 | 26.67 | 97.22 |
| Cmax (ng/mL) | 16 | 2.92 | 28.07 | 2.86 | 30.61 | 102.05 |
| tmax (h) | 16 | 6.88 | 19.06 | 7.81 | 25.70 | 87.98 |

**Table 10. PK data for tablets AML/CAN prepared according to Example 4; requirements 80 % <T/R<125%), reference: candesartan cilexetil 16 mg.**

| Parameter | N | TEST CAN-AML 16/10 mg Example 4 variant A | | REFERENCE CAN 16 mg | | RATIO |
|---|---|---|---|---|---|---|
| | | MEAN | CV (%) | MEAN | CV (%) | T/R (%) |
| AUC0-t (ng h/mL) | 16 | 1322.9 | 41.71 | 1265.0 | 42.05 | 104.58 |
| AUC0-∞ (ng h/mL) | 16 | 1466.8 | 43.53 | 1449.2 | 50.79 | 101.21 |
| Cmax (ng/mL) | 16 | 110.8 | 30.74 | 105.0 | 38.78 | 105.52 |
| tmax (h) | 16 | 5.72 | 26.80 | 5.63 | 33.96 | 101.59 |

**Table 11. PK data for tablets AML/CAN prepared according to Example 7; requirements 80 % <T/R<125%), reference: amlodipine 10 mg.**

| Parameter | N | TEST CAN-AML 16/10 mg CAN-AML 16/10 mg Example 7 | | REFERENCE AML 10 mg | | RATIO |
|---|---|---|---|---|---|---|
| | | MEAN | CV (%) | MEAN | CV (%) | T/R (%) |
| AUC0-t (ng h/mL) | 16 | 90.34 | 28.12 | 121.78 | 26.86 | 74.15 |
| AUC0-∞ (ng h/mL) | 16 | 161.50 | 20.99 | 170.19 | 26.67 | 94.89 |
| Cmax (ng/mL) | 16 | 1.92 | 27.01 | 2.77 | 30.61 | 69.31 |
| tmax (h) | 16 | 8.10 | 22.76 | 7.34 | 28.98 | 110.35 |

**Table 12. PK data for tablets AML/CAN prepared according to Example 7; requirements 80 % <T/R<125%), reference: candesartan cilexetil 16 mg.**

| Parameter | N | TEST CAN-AML 16/10 mg of Example 7 | | REFERENCE CAN 16 mg | | RATIO |
|---|---|---|---|---|---|---|
| | | MEAN | CV (%) | MEAN | CV (%) | T/R(%) |
| AUC0-t (ng h/mL) | 16 | 1412.9 | 42.22 | 1310.0 | 44.09 | 107.85 |
| AUC0-∞ (ng h/mL) | 16 | 1491.8 | 40.99 | 1550.2 | 45.90 | 96.23 |
| Cmax (ng/mL) | 16 | 108.2 | 33.08 | 105.0 | 40.13 | 103.05 |
| tmax (h) | 16 | 5.50 | 30.22 | 6.02 | 32.98 | 91.36 |

### Example 9

Dissolution testing was performed in following conditions (based on FDA-Recommended Dissolution Methods
Source:
http://www.accessdata.fda.gov/scripts/cder/dissolution/dsp_SearchResults_Di ssolutions.cfm.

| | Dissolution testing conditions for Candesartan Cilexetil | Dissolution testing conditions for Amlodipine |
|---|---|---|
| Medium: | 0.35% Polysorbate 20 in 0.05 M Phosphate Buffer | 0.1M HCl |
| RPM: | 50 | 75 |
| Volume: | 900 | 900 |
| Apparatus type: | 2 | 2 |

Dissolution studies were performed for tablets of AML/CAN prepared according to Example 4 variant A and Example 7 (comparative example). Results of the tests are presented in Tables 13-16.

**Table 13. Dissolution testing for tablets AML/CAN prepared according to Example 4 variant A, reference: candesartan cilexetil 16 mg.**

| DISSOLUTION DATA - 0.35% Polysorbate 20 in 0.05 M Phosphate Buffer | | | | | |
|---|---|---|---|---|---|
| CAN/AML tablets, 16/10 mg Example 4 variant A | | | CAN Reference tablets, 16 mg | | |
| Time point | Mean | RSD | Time point | Mean | RSD |
| 0 | 0.0 | - | 0 | 0.0 | - |
| 5 | 13.6 | 9.4 | 5 | 10.5 | 16.0 |
| 10 | 30.6 | 6.2 | 10 | 29.5 | 8.5 |
| 15 | 45.3 | 5.9 | 15 | 47.6 | 8.0 |
| 20 | 65.3 | 5.1 | 20 | 64.0 | 9.9 |
| 30 | 83.9 | 4.5 | 30 | 86.6 | 4.9 |
| 45 | 91.5 | 3.6 | 45 | 96.4 | 2.1 |
| 60 | 95.6 | 3.2 | 60 | 98.2 | 1.3 |
| inf | 99.1 | 0.8 | inf | 98.7 | 1.1 |

**Table 14. Dissolution testing for tablets AML/CAN prepared according to Example 4 variant A, reference: amlodipine 10 mg.**

| DISSOLUTION DATA - 0.1 M HCl | | | | | |
|---|---|---|---|---|---|
| CAN/AML tablets, 16/10 mg of Example 4 variant A | | | AML Reference tablets, 10 mg | | |
| Time point | Mean | RSD | Time point | Mean | RSD |
| 0 | 0.0 | - | 0 | 0.0 | - |
| 5 | 78.3 | 4.5 | 5 | 98.9 | 1.2 |
| 10 | 91.8 | 4.0 | 10 | 99.5 | 1.0 |
| 15 | 96.5 | 1.5 | 15 | 99.6 | 0.9 |
| 20 | 98.0 | 1.1 | 20 | 99.6 | 0.8 |
| 30 | 98.7 | 1.3 | 30 | 99.5 | 0.6 |
| inf | 99.0 | 1.3 | inf | 99.1 | 1.1 |

**Table 15. Dissolution testing for tablets AML/CAN prepared according to Example 7, reference: candesartan cilexetil 16 mg.**

| DISSOLUTION DATA - 0.35% Polysorbate 20 in 0.05 M Phosphate Buffer | | | | | |
|---|---|---|---|---|---|
| CAN/AML tablets, 16/10 mg Example 7 | | | CAN Reference tablets, 16 mg | | |
| Time point | Mean | RSD | Time point | Mean | RSD |
| 0 | 0.0 | - | 0 | 0.0 | - |
| 5 | 16.2 | 8.1 | 5 | 10.5 | 16.0 |
| 10 | 35.0 | 7.0 | 10 | 29.5 | 8.5 |
| 15 | 48.1 | 4.4 | 15 | 47.6 | 8.0 |
| 20 | 67.0 | 4.6 | 20 | 64.0 | 9.9 |
| 30 | 82.1 | 2.5 | 30 | 86.6 | 4.9 |
| 45 | 96.6 | 1.9 | 45 | 96.4 | 2.1 |
| 60 | 98.7 | 2.9 | 60 | 98.2 | 1.3 |
| inf | 100.7 | 1.3 | inf | 98.7 | 1.1 |

**Table 14. Dissolution testing for tablets AML/CAN prepared according to Example 7, reference: amlodipine 10 mg.**

| DISSOLUTION DATA - 0,1 M HCl | | | | | |
|---|---|---|---|---|---|
| CAN/AML tablets, 16/10 mg Example 7 | | | AML Reference tablets, 10 mg | | |
| Time point | Mean | RSD | Time point | Mean | RSD |
| 0 | 0.0 | - | 0 | 0.0 | - |
| 5 | 38.3 | 19.5 | 5 | 98.9 | 1.2 |
| 10 | 51.8 | 9.2 | 10 | 99.5 | 1.0 |
| 15 | 56.5 | 8.5 | 15 | 99.6 | 0.9 |
| 20 | 63.0 | 6.0 | 20 | 99.6 | 0.8 |
| 30 | 68.7 | 6.3 | 30 | 99.5 | 0.6 |
| inf | 97.7.0 | 3.0 | inf | 99.1 | 1.1 |

The results obtained for comparative study clearly show that in case of combination composition where the contact between active ingredients as well as between certain excipients is not limited - active ingredients are exposed to direct contact with factors that cause their degradation - and as a result the increase of impurities of both amlodipine and candesartan was observed. Namely, amlodipine contact with polyethylene glycol resulted in increased impurity D and discoloration of prepared tablets was observed as a result of o contact with lactose, which caused formation of colored impurities (Maillard reaction). Additionally, contact of candesartan with amlodipine salt (which has acidic character) results in transfer of ethyl group from benzimidazole ring to nitrogen atom in tetrazole ring and consequently in increased impurities B, C and D.

Stability testing under accelerated conditions for exemplary compositions shows that the pharmaceutical composition of the invention complies with the limits specified for the chemical purity of pharmaceutical preparations. The increase in impurities, which are degradation products of amlodipine and candesartan cilexetil is small and the product meets the requirements for medicinal products. It has been found that the pharmaceutical composition in unit dosage forms according to the invention exhibits an appropriate release profile comparable to the commercially available preparations containing respective single active ingredients. Furthermore, the process of the preparation of the composition and the dosage units of the invention is simple and economical.

The process for the preparation of a composition and the unit dosage forms does not require sophisticated equipment, needed for example for the manufacture of layered tablets. The process of preparing of the composition avoids exposing amlodipine to direct contact with factors that may cause its degradation: polyethylene glycol, which can result in increased impurity D, lactose, which may cause formation of colored impurities (Maillard reaction) and water, which may cause the hydrolysis of the ester groups in the amlodipine molecule.

Despite of the presence of polyethylene glycol or in some embodiments lactose in the granulate a), and therefore the possibility of contact of these excipients with amlodipine comprised in the extragranular phase b), enhanced level of impurities, which are degradation products of amlodipine, particularly the impurity D, was not observed. Thus the composition of the invention provides a safety of use of a combination pharmaceutical composition. Additionally, the low percentage of polyethylene glycol allows preparation of combination pharmaceutical composition which is stable and bioequivalent with the used active ingredients.

The results of bioequivalence studies show that lack of separation of active ingredients into two phases negatively influences bioequivalence of such prepared compositions with the appropriate referent active ingredients. On the opposite, the composition of the invention provides appropriate bioequivalence results. Thus the composition of the invention is superior over the known combination amlodipine/candesartan compositions stabilized with polyethylene glycols in which active ingredients are not separated into two phases.

Correlative data were obtained for in vitro studies: dissolution testing results clearly show that the dissolution profiles obtained for tablets prepared according to Example 7 are not consistent with the dissolution profiles of referent drugs compositions. In opposite - for the composition of Example 4 variant A dissolution studies accomplished for the combined composition of the invention prove excellent similarity to in vitro performance of both reference drugs.

Additionally, the use of two different types of hydroxypropylcellulose in form of a mixture allows to obtain the expected stability, release profiles and bioavailability of the combination composition of the invention, while reducing the number of process steps.

## Claims

1. A combination pharmaceutical composition in the form of a uniform solid blend consisting of:
a) a granulate comprising candesartan cilexetil, polyethylene glycol and excipients selected from the group consisting of a filler, a disintegrant, a binder and mixtures thereof, wherein the weight ratio of candesartan cilexetil to polyethylene glycol is in a range from 5.0:1.0 to 2.0:1.0, and
b) an extragranular phase which is a powder blend comprising amlodipine or a pharmaceutically acceptable salt thereof and excipients selected from the group consisting of a filler, lubricant and mixtures thereof, or from the group consisting of a disintegrant, a lubricant and mixtures thereof.

2. The pharmaceutical composition according to claim 1, wherein excipients in the granulate a) are a mixture comprising 83.0- 89.0%, preferably 85.0-90.0% by weight of the filler, 0.5 - 2.5%, preferably 1.0% or 2.0%; by weight of the disintegrant, and 5.0-8.0%, preferably 6.0 - 6.5% by weight of the binder, calculated on the total weight of the granulate a).

3. The pharmaceutical composition according to claim 1 or 2, wherein the filler is a mixture of lactose monohydrate and starch, and the weight ratio of candesartan cilexetil to polyethylene glycol is 4.8:1.0.

4. The pharmaceutical composition according to claim 1 or 2, wherein the filler is a mixture of lactose monohydrate and starch, and the weight ratio of candesartan cilexetil to polyethylene glycol is 2.4:1.0.

5. The pharmaceutical composition according to claim 1 or 2, wherein the filler is a mixture of starch, pregelatinized starch and microcrystalline cellulose, and the weight ratio of candesartan cilexetil to polyethylene glycol is 4.8:1.0 or 2.4:1.0.

6. The pharmaceutical composition according to any one of the preceding claims, wherein the disintegrant in the granulate a) is carboxymethylcellulose sodium salt, carboxymethyl starch sodium salt or carboxymethylcellulose calcium salt, preferably carboxymethylcellulose calcium salt.

7. The pharmaceutical composition according to any one of the preceding claims, wherein the binder in the granulate a) is hydroxypropylcellulose.

8. The pharmaceutical composition according to claim 7, wherein hydroxypropylcellulose is a mixture of hydroxypropylcellulose in the form of a solution and hydroxypropylcellulose in the form of a powder.

9. The pharmaceutical composition according to any one of the preceding claims, wherein the excipients in the extragranular phase b) are selected from the group consisting of a filler, a lubricant and mixtures thereof.

10. The pharmaceutical composition according to claim 9, wherein the excipients in the extragranular phase b) are a mixture of 80.0-95.0% by weight of the filler and 1.0-3.0% by weight of the lubricant.

11. The pharmaceutical composition according to claim 10, wherein the excipients in the extragranular phase b) are a mixture of 88.0% or 93% by weight of the filler and 1.0-3.0%, preferably 1.8 % by weight, of the lubricant.

12. The pharmaceutical composition according to any one of the claims 1 to 8, wherein the excipients in the extragranular phase b) are selected from the group consisting of the disintegrant, lubricant and mixtures thereof.

13. The pharmaceutical composition according to claim 12, wherein the excipients in the extragranular phase b) are a mixture of 22.0-66.0%, preferably 42.0-44.0% by weight of the disintegrant and 5.0-15.0 %, preferably 10.0-13.0% by weight of the lubricant.

14. The pharmaceutical composition according to any one of claims 9 to 11, wherein the filler in the extragranular phase b) is microcrystalline cellulose.

15. The pharmaceutical composition according to any one of claims 12 to 13, wherein the disintegrant in the extragranular phase b) is calcium salt of carboxymethylcellulose.

16. The pharmaceutical composition according to any one of claims 9 to 15, wherein the lubricant in the extragranular phase b) is sodium stearyl fumarate or magnesium stearate, preferably magnesium stearate.

17. The pharmaceutical composition according to any one of the preceding claims, wherein the weight ratio of the granulate a) to the extragranular phase b) is in a range from 1.0:1.0 to 2.0:1.0 or from 13.0:1.0 to 10.0:1.0.

18. The pharmaceutical composition according to claim 17, wherein the weight ratio of the granulate a) to the extragranular phase b) is 1.5:1.0 or 10.0:1.0 or 13.0:1.0.

19. A process for the preparation of a composition as defined in any of claims 1 to 18, consisting of the following steps:
- mixing the components of the granulate a) to obtain a homogeneous powder blend
- wet granulation of the homogeneous blend,
- drying the obtained granulate,
- uniformizing the granulate to form granulate a),
- mixing the obtained granulate a) with the components of the extragranular phase b).

20. A unit dosage form containing the pharmaceutical composition as defined in any one of claims 1 to 18 or prepared as defined in claim 19.

## Patentansprüche

1. Kombinierte pharmazeutische Zusammensetzung in Form von homogenes festes Blends, bestehend aus:
a) einem Granulat, enthaltend das Candesartancilexetil, Polyethylenglykol und Hilfsstoffe ausgewählt aus der Gruppe bestehend aus einem Füllstoff, einem Sprengmittel, einem Bindemittel und Gemischen davon, wobei das Gewichtsverhältnis von Candesartancilexetil zu Polyethylenglykol in einem Bereich von 5,0: 1,0 bis 2,0: 1,0 liegt, und
b) einer extragranulärer Phase, die ein Pulverblend ist, enthaltend die Amlodipin oder ein pharmazeutisch verträgliches Salz davon und Hilfsstoffe ausgewählt aus der Gruppe bestehend aus einem Füllstoff, einem Gleitmittel und Mischungen davon, oder aus der Gruppe bestehend aus einem Sprengmittel, einem Gleitmittel und Mischungen davon.

2. Pharmazeutische Zusammensetzung nach Anspruch 1, wobei die Hilfsstoffe in dem Granulat a) die Mischung sind, umfassend 83,0-89,0%, vorzugsweise 85,0-90,0 Gew .-% eines Füllstoffs, 0,5-2,5%, vorzugsweise 1,0% oder 2,0%, Gew .-% eines Sprengmittels und 5,0-8,0%, vorzugsweise 6,0-6,5 Gew .-% eines Bindemittels.

3. Pharmazeutische Zusammensetzung nach Anspruch 1 oder 2, wobei der Füllstoff eine Mischung von Lactosemonohydrat und Stärke ist und das Gewichtsverhältnis von Candesartancilexetil zu Polyethylenglycol 4,8: 1,0 beträgt.

4. Pharmazeutische Zusammensetzung nach Anspruch 1 oder 2, wobei der Füllstoff eine Mischung von Lactosemonohydrat und Stärke ist und das Gewichtsverhältnis von Candesartancilexetil zu Polyethylenglycol 2,4: 1,0 beträgt.

5. Pharmazeutische Zusammensetzung nach Anspruch 1 oder 2, wobei der Füllstoff eine Mischung von Stärke, vorgelatinierter Stärke und mikrokristalliner Cellulose ist und das Gewichtsverhältnis von Candesartancilexetil zu Polyethylenglykol 4,8: 1,0 oder 2,4: 1,0 beträgt.

6. Pharmazeutische Zusammensetzung nach einem der vorhergehenden Ansprüche, wobei das Sprengmittel in dem Granulat a) Carboxymethylcellulose-Natriumsalz, Carboxymethylstärke-Natriumsalz oder Carboxymethylcellulose-Calciumsalz, vorzugsweise Carboxymethylcellulose-Calciumsalz, ist.

7. Pharmazeutische Zusammensetzung nach einem der vorhergehenden Ansprüche, wobei das Bindemittel in dem Granulat a) Hydroxypropylcellulose ist.

8. Pharmazeutische Zusammensetzung nach Anspruch 7, wobei Hydroxypropylcellulose eine Mischung von Hydroxypropylcellulose in Form einer Lösung und Hydroxypropylcellulose in Form eines Pulvers ist.

9. Pharmazeutische Zusammensetzung nach einem der vorhergehenden Ansprüche, wobei die Hilfsstoffe in der extragranulärer Phase b) aus der Gruppe bestehend aus einem Füllstoff, einem Gleitmittel und Mischungen davon ausgewählt sind.

10. Pharmazeutische Zusammensetzung nach Anspruch 9, wobei Hilfsstoffe in der extragranulärer Phase b) eine Mischung von 80,0-95,0 Gew .-% dem Füllstoff und 1,0-3,0 Gew .-% des Gleitmittels sind.

11. Pharmazeutische Zusammensetzung nach Anspruch 10, wobei Hilfsstoffe in der extragranulärer Phase b) eine Mischung von 88,0 Gew .-% oder 93 Gew .-% dem Füllstoff und 1,0-3,0 Gew .-%, vorzugsweise 1,8 Gew .-%, des Gleitmittels sind.

12. Pharmazeutische Zusammensetzung nach einem der Ansprüche 1 bis 8, wobei Hilfsstoffe in der extragranulärer Phase b) aus der Gruppe bestehend aus einem Sprengmittel, einem Gleitmittel und Mischungen davon ausgewählt sind.

13. Pharmazeutische Zusammensetzung nach Anspruch 12, wobei die Hilfsstoffe in der extragranulärer Phase b) eine Mischung von 22,0-66,0 Gew .-%, vorzugsweise 42,0 - 44,0 Gew .-%, des Sprengmittels und 5,0 - 15,0 Gew .-%, vorzugsweise 10,0 - 13,0 Gew .-%, des Gleitmittels, sind.

14. Pharmazeutische Zusammensetzung nach einem der Ansprüche 9 bis 11, wobei der Füllstoff in der extragranulären Phase b) mikrokristalline Cellulose ist.

15. Pharmazeutische Zusammensetzung nach einem der Ansprüche 12 bis 13, wobei das Sprengmittel in der extragranulären Phase b) Calciumsalz von Carboxymethylcellulose ist.

16. Pharmazeutische Zusammensetzung nach einem der Ansprüche 9 bis 15, wobei das Gleitmittel in der extragranulären Phase b) Natriumstearylfumarat oder Magnesiumstearat, vorzugsweise Magnesiumstearat, ist.

17. Pharmazeutische Zusammensetzung nach einem der vorhergehenden Ansprüche, wobei das Gewichtsverhältnis des Granulats a) zur extragranularen Phase b) in einem Bereich von 1,0: 1,0 bis 2,0: 1,0 oder von 13,0: 1.0 bis 10,0: 1,0 liegt.

18. Pharmazeutische Zusammensetzung nach Anspruch 17, wobei das Gewichtsverhältnis des Granulats a) zur extragranularen Phase b) 1,5: 1,0 oder 10,0: 1,0 oder 13,0: 1, 0 beträgt.

19. Verfahren zur Herstellung von Zusammensetzung nach einem der Ansprüche 1 bis 18, bestehend aus den folgenden Schritten:
- Mischung der Komponenten des Granulats a), um eine homogene Pulverblend zu erhalten
- Feuchtgranulation der homogenen Blend,
- Trocknung der erhaltenen Mischung Granulat,
- Homogenisierung des Granulats um das Granulat a) zu erhalten
- Mischung des erhaltenen Granulats a) mit den Komponenten der extragranularen Phase b).

20. Einheitsdosierungsform, enthaltend die pharmazeutische Zusammensetzung nach einem der Ansprüche 1 bis 18 oder hergestellt nach Anspruch 19.

## Revendications

1. Composition pharmaceutique combinée sous forme de mélange uniforme solide, consistant en:
a) un granulat comprenant candésartan cilexétil, polyéthylèneglycol et des excipients choisis dans le groupe constitué d'un diluant, d'un delitant, d'un liant et de mélanges de ceux-ci, dans lequel le rapport pondéral du candésartan cilexétil au polyéthylèneglycol est compris entre 5,0: 1 et 2,0: 1, et
b) une phase extragranulaire qui est un mélange de poudres comprenant de l'amlodipine ou un de ses sels pharmaceutiquement acceptables et des excipients choisis dans le groupe constitué d'un diluant, d'un lubrifiant et de mélanges de ceux-ci, ou dans le groupe constitué d'un délitant, d'un lubrifiant et de mélanges de ceux-ci.

2. Composition pharmaceutique selon la revendication 1, dans laquelle les excipients dans le granulat a) sont un mélange comprenant 83,0-89,0%, de préférence 85,0-90,0% en poids d'un diluant, 0,5-2,5%, de préférence 1,0% ou 2,0% en poids d'un délitant, et 5,0-8,0%, de préférence 6,0 - 6,5% en poids d'un liant, calculé sur le poids total du granulat a).

3. Composition pharmaceutique selon la revendication 1 ou 2, dans laquelle le diluant est une mélange de lactose monohydraté et d'amidon, et le rapport pondéral du candésartan cilexétil au polyéthylèneglycol est de 4,8: 1,0.

4. Composition pharmaceutique selon la revendication 1 ou 2, dans laquelle le diluant est une mélange de lactose monohydraté et d'amidon, et le rapport pondéral du candésartan cilexétil au polyéthylèneglycol est de 2,4: 1,0.

5. Composition pharmaceutique selon la revendication 1 ou 2, dans laquelle le diluant est une mélange d'amidon, d'amidon prégélatinisé et de cellulose microcristalline, et le rapport pondéral du candésartan cilexétil au polyéthylèneglycol est de 4,8: 1,0 ou 2,4: 1,0.

6. Composition pharmaceutique selon l'une quelconque des revendications précédentes, dans laquelle le delitant dans le granulat a) est un sel de sodium de carboxyméthylcellulose, un sel de carboxyméthylamidon sodique ou un sel de calcium de carboxyméthylcellulose, de préférence un sel de calcium de carboxyméthylcellulose.

7. Composition pharmaceutique selon l'une quelconque des revendications précédentes, dans laquelle le liant dans le granulat a) est l'hydroxypropylcellulose.

8. Composition pharmaceutique selon la revendication 7, dans laquelle l'hydroxypropylcellulose est un mélange d'hydroxypropylcellulose sous forme de solution et d'hydroxypropylcellulose sous forme de poudre.

9. Composition pharmaceutique selon l'une quelconque des revendications précédentes, dans laquelle les excipients dans une phase extragranulaire b) sont choisis dans le groupe constitué d'un diluant, d'un lubrifiant et de mélanges de ceux-ci.

10. Composition pharmaceutique selon la revendication 9, dans laquelle les excipients dans une phase extragranulaire b) sont une mélange de 80,0-95,0% en poids d'un diluant et 1,0-3,0% en poids d'un lubrifiant.

11. Composition pharmaceutique selon la revendication 10, dans laquelle les excipients dans une phase extragranulaire b) sont une mélange de 88,0 ou 93% en poids d'un diluant et 1,0-3,0%, de préférence 1,8%, en poids d'un lubrifiant.

12. Composition pharmaceutique selon l'une quelconque des revendications 1 à 8, dans laquelle les excipients dans une phase extragranulaire b) sont choisis dans le groupe constitué d'un delitant, d'un lubrifiant et de mélanges de ceux-ci.

13. Composition pharmaceutique selon la revendication 12, dans laquelle les excipients dans une phase extragranulaire b) sont une une mélange de 22,0-66,0%, de préférence 42,0-44,0% en poids d'un delitant et 5,0-15,0 %, de préférence 10,0-13,0% en poids d'un lubrifiant.

14. Composition pharmaceutique selon l'une quelconque des revendications 9 à 11, dans laquelle le diluant dans une phase extragranulaire b) est la cellulose microcristalline.

15. Composition pharmaceutique selon l'une quelconque des revendications 12 à 13, dans laquelle le delitant dans une phase extragranulaire b) est la sel de calcium de la carboxyméthylcellulose.

16. Composition pharmaceutique selon l'une quelconque des revendications 9 à 15, dans laquelle le lubrifiant dans une phase extragranulaire b) est stéarylfumarate de sodium ou le stéarate de magnésium, de préférence le stéarate de magnésium.

17. Composition pharmaceutique selon l'une quelconque des revendications précédentes, dans laquelle le rapport pondéral du granulat a) à la phase extragranulaire b) est compris dans l'intervalle de 1,0: 1,0 à 2,0: 2,0 ou de 13,0: 1,0 à 10,0: 1.0.

18. Composition pharmaceutique selon la revendication 17, dans laquelle e rapport pondéral du granulat a) à la phase extragranulaire b) est 1,5: 1,0 ou 10,0: 1,0 ou 13,0: 1,0.

19. Procédé pour la préparation d'une composition selon l'une quelconque des revendications 1 à 18, consistant d'étapes suivantes:
- mélanger des composants du granulat a) pour obtenir un mélange homogène de poudres
- granulation humide du mélange homogène,
- séchage du granulat obtenu,
- uniformiser le granulat pour former le granulat a),
- mélanger le granulat a) obtenu avec les composants de la phase extragranulaire b).

20. Forme de dosage unitaire contenant la composition pharmaceutique telle que définie dans l'une quelconque des revendications 1 à 18 ou préparée comme défini dans la revendication 19.
